# EUROPEAN PATENT APPLICATION

(11) **EP 4 606 358 A1**
(43) Date of publication of application: **27.08.2025**
(21) Application number: 24159408.4
(22) Date of filing: 23.02.2024
(51) Int. Cl.: A61F 7/12, A61B 18/04, A61B 18/00, A61M 25/00, A61F 7/00

(54) **LIQUID INJECTION DEVICE**

(71) Applicant: Schelin Medicin AB, 39395 Rockneby (SE)
(72) Inventor: SONNY, Schelin, 380 30 Rockneby (SE)
(74) Representative: Hansson Thyresson AB

(57) **Abstract**

There is provided a liquid injection device (10) comprising an injection tube (12), wherein the injection tube (12) extends in a hollow tip (14) with a plurality of openings (16). At least three circumferentially displaced openings (16) are provided around the hollow tip (14) below an edge (15) of the hollow tip (14) and the hollow tip (14) is provided with a non-cutting edge. There is also provided a method for performing heat therapy of mammalian tissue, comprising the steps: inserting an injection tube (12) extending in a hollow tip (14) in a selected position in the tissue, injecting a liquid including at least one anesthetic and adrenaline heated to a temperature of at least 37° C through the injection tube and through a plurality of openings (16) below an edge (15) of the hollow tip (14) into the mammalian tissue.

## Description

### TECHNICAL FIELD

The present invention relates to a liquid injection device, in particular a liquid injection device comprising a hollow tip with a plurality of openings, and a method of operating the same for performing heat therapy.

### BACKGROUND

Hyperthermia is an adjuvant local anti-cancer treatment using temperatures exceeding the physiologically optimal level, typically below 45 °C. Hyperthermia treatment (<45°C) of cancer diseases is well studied and well documented. Adjuvant hyperthermia treatment enhances the effect of radiotherapy, chemotherapy, and immunotherapy, specifically in different solid cancers and prostate cancer."

Hyperthermia applied as radiosensitizer or chemosensitizer has shown great results in over four decades and is presently successfully applied in combination with radiotherapy or chemotherapy for treatment of many tumour types, including recurrent breast cancer, bladder cancer, cervical carcinoma, head & neck cancer, soft tissue sarcoma, and melanoma.

Hyperthermia affects cells and tissues in various ways. It can directly alter the physical properties of cellular components, but it can also influence cellular responses. Hyperthermia affects multiple intracellular processes, like e.g., DNA repair pathways, as well as systemic immune responses. Hyperthermia can also modify factors that are essential for tumour survival and growth, such as the microenvironment, immune responses, vascularisation, and oxygen supply. Thus, the effects of hyperthermia are multifactorial, as addressed in the contributions in this issue. Hyperthermia inhibits the homologous recombination (HR) DNA repair pathway by inducing proteasomal degradation of for instance BRCA2.

The effectiveness of hyperthermia as a treatment modality for cancer is continuously improved. One of the disappointing findings has been the inability to deliver uniform thermal doses to tumor volumes. This inability to heat certain tumors is due to a variety of physical and physiologic phenomena. To increase the ability of heating tumors, local interstitial techniques have been developed that are proving to be safe and effective. These techniques employ implanted microwave or radiofrequency antennae for the delivery of local thermal doses. Another technique involves the placement of interstitially located ferromagnetic seeds for local hyperthermia have also been conducted. The seeds can be heated by delivery of a high-wattage RF magnetic field to the implanted volume by an external source after implantation. The tissue surrounding the ferromagnetic implant is heated by conduction of heat away from the implanted seeds. Achievement of an effective treatment requires high quality heating equipment, precise thermal dosimetry, and adequate quality assurance. Several types of devices, antennas and heating or power delivery systems have been proposed and developed in recent decades. These vary considerably in technique, heating depth, ability to focus, and in the size of the heating focus.

From the above it is understood that there is room for improvements and the invention aims to solve or at least mitigate the above and other problems.

### SUMMARY

A way of improving the effectiveness of hyperthermia as a treatment modality for cancer would be to provide a device that will deliver uniform thermal doses to tumor volumes. This may comprise providing an efficient spreading of a heating liquid in the tissue comprising cancer cells. Also noncancerous conditions, such as benign prostate enlargement (BPE), also known as benign prostatic hyperplasia (BPH), can be treated. The main difference between treating cancer and noncancerous conditions will be the temperature of the thermal doses. Cancer cells are more sensitive to increased temperatures than other cells. Thus, lower temperatures can be used for treatment of cancer. In cancer treatments, an appropriate temperature of treatment can be in the interval of 39° and 60° Celsius. For treatment of noncancerous conditions, such as benign prostatic hyperplasia (BPH), the temperature of treatment can be in the interval of 45° and 70° Celsius, or even higher.

The energy transferred to the tissue is dependent on the mass of the tissue and is calculated on the basis of the temperature and the volume of the liquid. It is possible to use the Penne's bioheat transfer equation and the Henrique's burn integral equation for the calculations and determination of the required energy.

A suitable liquid injection device comprises an injection tube, wherein the injection tube extends in a needle or hollow tip with a plurality of openings. The injection tube is also referred to as a cannula. The needle or hollow tip is be provided with a non-cutting edge point. The openings are displaced around the hollow tip to allow spreading of uniform thermal doses at appropriate temperatures to tumor or noncancerous cell volumes where the hollow tip has been inserted. The needle is penetrating tissue displacing it when introduced and the tissue is returning to its original position when the needle is retracted. The bleeding risk is minimized while the penetration space is closed by tissue elasticity. The liquid injection device can be used in combination with ongoing anticoagulant medications.

The invention is defined by the appended independent claims. Additional features and advantages of the concepts disclosed herein are set forth in the description which follows, and in part will be obvious from the description, or may be learned by practice of the described technologies. The features and advantages of the concepts may be realized and obtained by means of the instruments and combinations particularly pointed out in the appended claims. These and other features of the described technologies will become more fully apparent from the following description and appended claims, or may be learned by the practice of the disclosed concepts as set forth herein.

In a first aspect the disclosed liquid injection device comprises an injection tube, wherein the injection tube extends in a hollow tip with a plurality of openings. At least three circumferentially displaced openings are provided around the hollow tip below an edge of the hollow tip. The hollow tip is provided with a non-cutting edge.

In a second aspect there is disclosed a method for performing heat therapy of mammalian tissue, the method comprising the steps: (a) inserting an injection tube extending in a hollow tip in a selected position in the tissue, and (b) injecting a liquid including at least one anesthetic and adrenaline heated to a temperature of at least 37° C through the injection tube and through a plurality of openings below an edge of the hollow tip into the mammalian tissue.

### BRIEF DESCRIPTION OF THE DRAWINGS

In order to best describe the manner in which the above-described embodiments are implemented, as well as define other advantages and features of the disclosure, a more particular description is provided below and is illustrated in the appended drawings. Understanding that these drawings depict only exemplary embodiments of the invention and are not therefore to be considered to be limiting in scope, the examples will be described and explained with additional specificity and detail through the use of the accompanying drawings in which:
- Fig. 1: is a schematic perspective view of the disclosed liquid injection device,
- Fig. 2a-c: are schematic side elevation views of different embodiments of a hollow tip of the liquid injection device as shown in Fig. 1,
- Fig. 3: is a cross sectional view from line X-X in Fig. 2a,
- Fig. 4: is a schematic perspective view of an embodiment of the disclosed liquid injection device in combination with a treatment catheter,
- Fig. 5: is a side sectional view of one embodiment of the disclosed liquid injection device in position in a prostate, and
- Fig. 6: is a schematic perspective view of an embodiment of the disclosed liquid injection device in combination with an introduction unit.

Further, in the figures like reference characters designate like or corresponding parts throughout the several figures.

### DETAILED DESCRIPTION

Various embodiments of the disclosed methods and arrangements are discussed in detail below. While specific implementations are discussed, it should be understood that this is done for illustration purposes only. A person skilled in the relevant art will recognize that other components, configurations, and steps may be used without parting from the spirit and scope of the disclosure. In the description and claims the word "comprise" and variations of the word, such as "comprising" and "comprises", does not exclude other elements or steps.

Hereinafter, certain embodiments will be described more fully with reference to the accompanying drawings. It will be apparent to those skilled in the art that various modifications and variations can be made without departing from the inventive concept. Other embodiments will be apparent to those skilled in the art from consideration of the specification and practice disclosed herein. The embodiments herein are provided by way of example so that this disclosure will be thorough and complete and will fully convey the scope of the inventive concept, and that the claims be construed as encompassing all equivalents of the present inventive concept which are apparent to those skilled in the art to which the inventive concept pertains. If nothing else is stated, different embodiments may be combined with each other.

A liquid injection device 10 will be described below with reference to the figures.

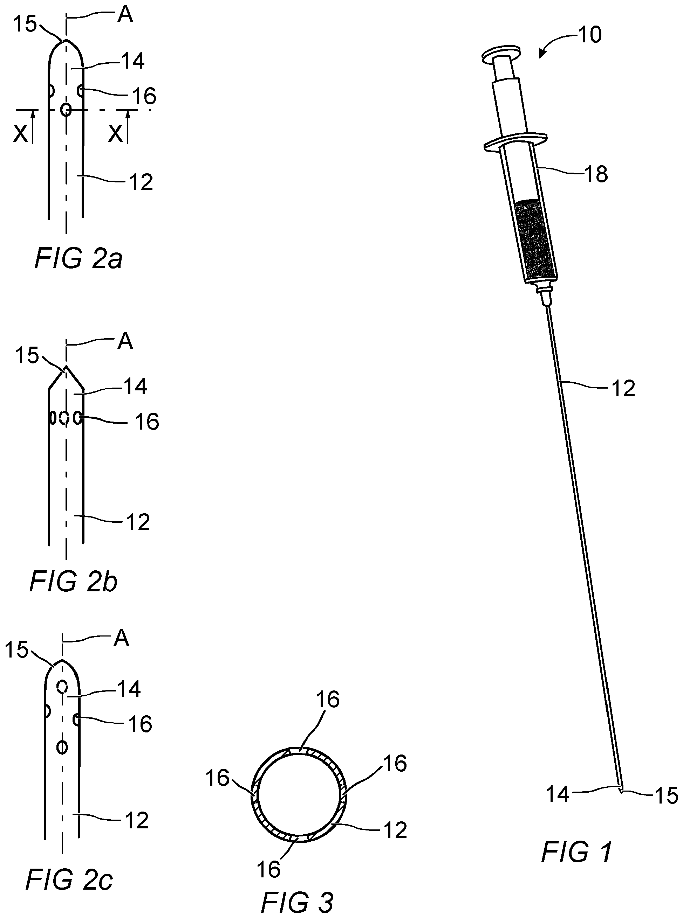

The disclosed liquid injection device 10 comprises a longitudinal injection tube or cannula 12 extending in a hollow tip 14 with a plurality of openings 16. The hollow tip 14 is elongated and extends along an axis A. The injection tube 12 and the hollow tip 14 may be provided as one single unit, the hollow tip being a final part of the injection tube. The openings 16, as shown in Fig. 2a to Fig. 2c are circumferentially displaced around the hollow tip 14. This is shown also in Fig. 3. A liquid injected through the openings 16 will spread the liquid in the tissue where the hollow tip 14 is inserted. By providing the openings 16 circumferentially evenly around the periphery of the hollow tip the liquid will be spread optimally and in a uniform way in the tissue around the hollow tip 14. For various tissues, the diameter of the injection tube 12 can be in the interval 1mm to 1,4mm. A diameter of the injection tube 12 for prostate tissue can be 1,2mm.

A liquid ejector 18 is used for injecting the fluid into the injection tube 12. In the embodiment shown in Fig. 1, the liquid ejector 18 is a conventional syringe. In various embodiments, the liquid ejector 18 is an apparatus comprising a pump or similar device for administration of a liquid.

In the embodiment shown in Fig. 2a, there are four openings 16 circumferentially and evenly displaced around the hollow tip 14. The four openings 16 are provided two by two diametrically opposite to each other. A first set of two of the diametrically opposite openings are provided axially displaced from a second set of two of the diametrically opposite openings.

In the embodiment shown in Fig. 2b, there are three openings 16 circumferentially and evenly displaced around the hollow tip 14. Thus, the three openings 16 are displaced 120° from each other. All three are provided at the same position in the axial direction.

In the embodiment shown in Fig. 2c, there are four openings 16 circumferentially and evenly displaced around the hollow tip 14. The four openings 16 are provided two by two diametrically opposite to each other. All four openings 16 are displaced in the axial direction.

In various embodiments, other configurations of the openings 16 are selected with respect to the strength of the material used in the hollow tip 14. The size of the openings may also vary in dependence of the strength of the material used in the hollow tip 14 and of an appropriate operating flow of the liquid. The hollow tip 14 is provided with a non-cutting edge or point 15. The point 15 can be rounded, as shown in Fig. 2a and Fig. 2c, or sharper as shown in Fig. 2b.

The disclosed liquid injection device 10 can be used in a method for performing heat therapy or hyperthermia of mammalian tissue. The injection tube 12 is inserted in a selected position in the tissue. A fluid heated to an appropriate temperature is injected through the injection tube 12 and the hollow tip 14 into a portion of the mammalian tissue. The heated fluid exits the hollow tip 14 through the plurality of openings 16 to be spread appropriately in the tissue. The fluid is heated to a temperature that is sufficiently high to ensure that the temperature of the liquid will be 37° C or higher than when it is spread in the tissue. Heat from the heated fluid will be transferred as a convective heat transfer to the tissue. Some heat will be transferred by conduction.

In some circumstances, several injections are required to heat a selected volume of the tissue. In various embodiments, the injection tube is moved in an axial direction between different positions within the tissue, and the heated liquid is injected at different positions. When larger tissue sections are selected for treatment, the injection tube is removed completely from the tissue and then introduced at another position in the tissue. In at least some of the injections, the liquid includes at least one local anesthetic substance and Adrenaline.

By injecting Adrenaline, the flow of blood is reduced or even blocked out in the selected tissue. As a result, the cooling effect of the blood in the tissue is also reduced or blocked.

The disclosed method of hyperthermia can be administrated together with different kinds of medicine - single or repeated injections or combinations of neoadjuvant or adjuvant medicine as a multimodal treatment at different temperatures. The temperature exposure by fluid convection can vary between 37° and 70° Celsius.

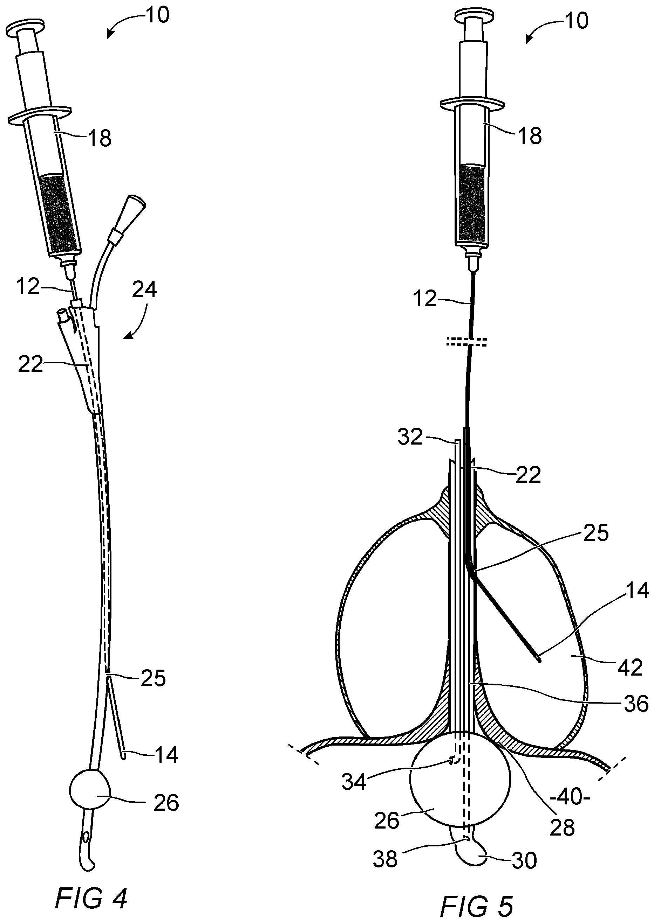

The disclosed method involves the following steps when used in a treatment catheter 24, as shown in Fig. 4 and Fig. 5, using examples of heated and other liquids.

The treatment catheter 24 is introduced through urethra, inflated to 20 ml with sterile water, rotated to the 5 o'clock position according to the external visual line, and pulled down until a balloon 26 of the treatment catheter 24 rests against the bladder neck 28. An end tip 30 of the treatment catheter 24 extends into the bladder 40. The treatment catheter 25 is further provided with an internal first tube 32 for inflation of the ballon. The first tube 32 ends with a first tube opening 34 inside the bal-Ion. The treatment catheter 25 is further provided with an internal second tube 36 for drainage of the bladder. The second tube 36 ends with a second tube opening 38 that will be positioned inside the bladder for draining the bladder.

The cannula 12 is then inserted to its depth through the catheter 24 with the hollow tip 14 in position in the prostate tissue 42 extending from an outlet opening 25 in the catheter. After aspiration, if clear of blood, 1 ml of 0.5% Carbocaine Adrenaline heated to the appropriate temperature is injected slowly. When there is bloody aspirate or an increase in pulse, the position of the hollow tip 14 of the cannula 12 is changed or repositioned. The cannula's position before injection can be checked via transrectal ultrasound if necessary. After 1 minute, following a new negative aspiration, an additional 2 ml is injected forcefully, 1 ml at a time. The cannula 12 is retracted by 1 cm, and another 2 ml is infiltrated in the same manner through the hollow tip 14. The cannula is again retracted by 1 cm, and an additional 2 ml is infiltrated. The same procedure is repeated after rotating the catheter, for instance in the following order: at 7 o'clock, 11 o'clock, 1 o'clock, 3 o'clock, and 9 o'clock. A total of 40 ml is injected. Depending on the prostate size, each injection position may be supplemented with additional amounts of sterile physiological saline heated to the appropriate temperature immediately after the above injections and before moving the cannula to a new position. The treatment catheter could be produced in different versions with the hollow tip or needle leaving the treatment catheter in different distances from the balloon to reach closer to the peripheral zone, harboring prostate cancer, adjusted and produced to be positioned centrally in relation to the actual prostate volume. The free needle part - length inside the prostate varies but the tip position, when fully introduced, will relate to the balloon in the bladder neck and thereby to the entrance of blood vessels and nerves blocking blood flow and reaching full prostate anesthesia. Hyperthermia could be guided to the perfect clockwise needle position by help of the magnetic resonance imaging, MRI, to picture fusion in the ultrasound image to apply neoadjuvant or adjuvant hyperthermia plus local anaesthesia and Adrenaline. If there is a pronounced median lobe, injections of hyperthermic fluid up to 70°, for example including Mepivacain Adrenaline (MA) into that lobe 6 o'clock to induce necrosis could complement Transurethral microwave thermotherapy (TUMT) treatment effects on the sidelobes.

Infiltrations of hot MA or physiologic NaCl or combinations could induce thermotherapy and tissue necrosis centrally along the infiltration canal in different clockwise positions killing BPH tissue symmetrically enough to resolve a BPH obstruction. It can functionate as a new attractive easy, pain-free alternative minimally invasive outpatient thermotherapy for all prostate sizes. Mucosal coating in the prostatic urethra will stay intact and alive and prevent surface necrosis avoiding posttreatment tissue sloughing in infection risk. Additional partial or clockwise treatment could be administered to asymmetric rest adenoma if needed.

For corresponding injections via cystoscope and Albarran bridge, they should be given in the same manner and order. No stick bleeding disrupts the view through the cystoscope thanks to the non-cutting edge of the hollow tip 14.

Pre-filled sterile syringes in a sterile glove can be heated in water in heat containers with an adjustable thermostat.

In various embodiments, Adrenaline in combination with a local anesthesia, such as Mepivacaine, Lidocaine, or Bupivacaine, is included in the heated liquid. An appropriate combination is Mepivacain Adrenaline 0,5%. Also other kinds of local anaesthesia + Adrenaline of different concentrations can be used alone or in combination with complementing dose/response volumes of physiologic NaCl at different temperatures. The injected fluid may also be hypo- or hyperosmolar solutions and may contain "slow release" pharmaceutical preparations for example antibiotics, cortisone, hormones, anti-hormones, cytostatic medicines, immunologic locally active treatments, or combinations. Local medicine release may reduce or minimize general systemic side effects. Biophysical calculations on administrated heat energy preferably should be made in relation to targeted tissue or cancer volume. The basis for such calculations can for instance be the Arrhenius equation or complements. Temperature feedback from the tissue exposed to the heat treatment could improve the treatment effectiveness and can be used in combination with or as a complement to the biophysical calculations.

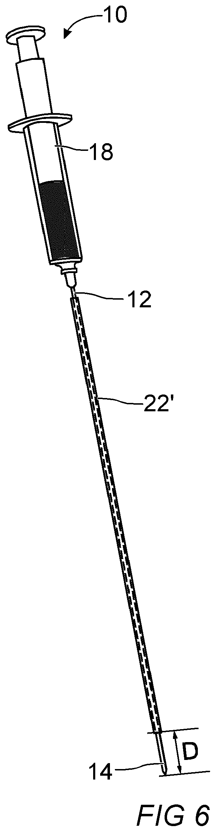

The injection tube 12 can be used in different applications, such as for instance independently as shown in Fig. 1, mounted in a in an introduction unit 22 in treatment catheter 24 as shown in Fig. 4 and Fig. 5, or extending in a hollow tube 22', as shown in Fig. 6. In the first and latter case, it can be used for treatment of solid tumours in different tissues in the mammalian or human body and should preferably be stiff and ridged to be introduced per se. When used for the treatment of solid tumours the point 15 preferably is rather sharp, as shown in Fig. 2b.

When used in a treatment catheter 24 for a combination with heat treatment of prostate tissue, such as for the treatment of BPH, the injection tube 12 and hollow tip 14 are provided in an introduction unit 22, such as a guide tube, and can be made of PEEK (polyether ether ketone). Again, suitably the injection tube and hollow tip are provided as one single unit, and where the hollow tip is only the final part of that single unit. By injecting Adrenaline in selected prostate base areas and the flow of blood is reduced or even blocked out just before heating the selected tissue or the whole prostate. As a result, the cooling effect of the blood in the tissue is also reduced or blocked.

To be able to locate the position of the hollow tip may be important when performing a prostate surgery method. The hollow tip is the final part of the injection tube which is possible to extend from the introduction unit 22 and thus possible to introduce into the prostate. Furthermore, according to one embodiment of the present invention the hollow tip arranged to be extended from the introduction unit is arranged to extend from the introduction unit with an angle in the range of 20°-40° from the introduction unit. This angle is suitable angle for introducing the hollow tip into the prostate. The positioning of the treatment catheter when the hollow tip is about to be introduced into the prostate should be well determined. To enable this the choice of material can be important. A material possible to observe when using assisting technologies, such as ultrasound, CT etc., during surgery may be of interest in various embodiments. At the same time, the material of the hollow tip and injection tube may be suitable for introducing into the prostate. PEEK is one such suitable material. Furthermore, the injection tube may be reinforced, such as by use of a steel material.

Furthermore, as the injection tube and hollow tip is made in one piece and of a suitable (stiff but flexible) material, e.g. PEEK, this provides for the ability to remove the hollow tip from one site of the prostate to insert it at another site of the prostate in a subsequent step. Furthermore, in relation to the material of the injection tube, this should be stiff enough but still flexible, without deforming when being bended at an exit position of the treatment catheter. If the injection tube 12 is mounted in a treatment catheter for treatment of prostate it can be left in place as an ordinary indwelling catheter for minutes, hours, days, or weeks during repeated intraprostatic treatments. Treatment indications for use in a treatment catheter could be prostate cancer, BPH, and prostatitis in different settings and combinations.

If used together with cystoscopy to infiltrate the prostate by help of an "Albarran bridge" device it should preferably tolerate a soft deviation angle of 30°.

The disclosed hyperthermia method preferably is used in solid tumors in appropriate locations, such as against prostate cancer or other solid cancers. The reduction of the flow of blood in the treated tissue due to the injection of adrenaline will also result in hypoxia and a lower risk of bleeding. Furthermore, the heating of the tissue in combination with the hypoxia will cause a rapidly increasing anaerobic metabolism that will cause a pronounced lactic acidosis. Scientific studies show that hypoxia and resulting lactic acidosis will increase the sensibility to heat of cancer cells. Repeated injections will extend the period of hypoxia and increase a transfer of heat and thus further improve the heat therapy.

The heat sensitivity of benign prostate cells is in vitro documented (Bowmic et al.). In a well-established thermotherapy, such as disclosed for instance in US 6,584,361, microwaves kill benign and malignant cells in the prostate. Heat is spread through conduction from an electromagnetic radiating antenna positioned in the prostate's urethra, operating at 915 MHz with a penetration depth of <15 mm in human tissue.

Both cell breakdown during necrosis, exposing cytoplasmic organelles and cell nucleus antigens, or after apoptotic cell death due to hyperthermia, are considered to initiate an immunological activation.

This concept is a well-established treatment model where an unpublished Swedish registry study shows a significantly reduced incidence and mortality of prostate cancer after such a treatment as compared to TURP operations.

The use of the disclosed method and device may imply a kind of "vaccination against one's own cancer".

In the embodiment shown in Fig. 6, the injection tube 12 extends in an alternative embodiment of the introduction unit 22 that is formed as a more rigid socket or hollow tube 22'. The hollow tube 22' is sufficiently stiff to be inserted into a selected tissue and close to the tissue selected for heat treatment. An extending section of the hollow tip 14 extends from the hollow tube. The extending section have a length D, which may depend on the tissue where the hollow tube 22' and hollow tip 14 are inserted. The length D can be approximately 50 mm or less. The hollow tube 22' prevents a fracture of the injection tube 14, and hindrance to penetrate sufficiently deep to reach a target position in the selected tissue. The diameter of the hollow tube 22' is somewhat larger than the diameter of the injection tube 12. In various embodiments, the larger diameter of the hollow tube 22' allows an axial movement of the injection tube 12 inside the hollow tube 22'.

Heat therapy is frequently used and well-known since many years to reduce pain related to prostatitis. This could be a new, easy, pain-free, quick, sterile, and safe way to help severe cases with prostatitis induced chronic pain. Prolonged exposure to heat, hypoxia, and lactic acidosis in the target area can be achieved by leaving the needle in place and repeated infiltrations with Adrenaline and warm fluid every 10 minutes. Adrenaline effect will block blood flow for longer time and acidosis will be even more pronounced. Injected fluid may be acid per se.

Different combinations of treatments are possible, such as neoadjuvant hyperthermia plus local anaesthesia and Adrenaline together with different kinds of radiotherapy, cytostatic-, immunologic treatments and with local electric or ultrasound prostate cancer treatments. It is also possible to use adjuvant hyperthermia plus local anaesthesia and Adrenaline together with different kinds of radiotherapy, cytostatic-, immunologic treatments and with local electric or ultrasound prostate cancer treatments. It could also be used neoadjuvant or adjuvant together with microwave treatments or other minimally invasive treatments for BPH.

As indicated above different temperature intervals of the liquid preferably should be used for different treatment indications.
- Hyperthermia 37° - 45° against chronic prostatitis pain,
- Hyperthermia 41° - 45° for incidental prostate cancer or adjuvant other treatments,
- Thermotherapy 45° - 70° for BPH/ Benign prostatic obstruction (BPO) and prostate cancer.

The various embodiments described above are provided by way of illustration only and should not be construed to limit the invention. For example, the principles herein may be applied to any device and method for treatment of cancer. Those skilled in the art will readily recognize various modifications and changes that may be made to the present invention without following the example embodiments and applications illustrated and described herein, and without departing from the scope of the present disclosure.

## Claims

1. A liquid injection device (10) comprising an injection tube (12), wherein the injection tube (12) extends in a hollow tip (14) with a plurality of openings (16);
wherein at least three circumferentially displaced openings (16) are provided around the hollow tip (14) below an edge (15) of the hollow tip (14); and
wherein the hollow tip (14) is provided with a non-cutting edge.

2. The liquid injection device (10) as claimed in claim 1, wherein the at least three circumferentially displaced openings (16) are provided evenly circumferentially displaced.

3. The liquid injection device (10) as claimed in claim 1 or claim 2, wherein an injection connection is connected to the injection tube and wherein the injection connection is configured to receive a liquid ejector.

4. The liquid injection device (10) as claimed in claim 1 or claim 2, wherein an injection connection is connected to the injection tube and wherein the injection connection is connected to a liquid ejector; and wherein the liquid ejector contains a liquid heated to a temperature between 37° C and 70° C.

5. The liquid injection device (10) as claimed in claim 3 or 4, wherein the liquid ejector is connected to the injection connection and comprises a syringe.

6. The liquid injection device (10) as claimed in any of the preceding claims, wherein the injection tube (12) extends in a hollow tube (22').

7. The liquid injection device (10) as claimed in any of claims 1-5, wherein the injection tube (12) is provided in a treatment catheter (24) and extendable from an outlet opening (25) of said treatment catheter (24).

8. A method for performing heat therapy of mammalian tissue, comprising
a) inserting an injection tube (12) extending in a hollow tip (14) in a selected position in the tissue,
b) injecting a liquid including at least one anesthetic and adrenaline heated to a temperature of at least 37° C through the injection tube and through a plurality of openings (16) below an edge (15) of the hollow tip (14) into the mammalian tissue.

9. The method as claimed in claim 8, further comprising
c) axially displacing the hollow tip (14) to another selected position in the tissue,
d) injecting a liquid including at least one anesthetic and adrenaline heated to a temperature of at least 37° C through the injection tube and through a plurality of openings (16) below an edge (15) of the hollow tip (14) into the mammalian tissue, and
e) repeating steps c) and d) at least twice.

10. The method as claimed in claim 8, wherein the liquid is a solution of Mepivacaine Adrenaline 0,5%.

11. The method as claimed in claim 8, further comprising after steps a) and b) injecting sterile sodium chloride heated to a temperature of at least 39° C through the injection tube and through openings in the hollow tip into the mammalian tissue.

12. The method as claimed in claim 8, wherein the mammalian tissue is prostate tissue, further comprising:
a) inserting a catheter (24) through the urethra to a position where an end tip (30) and a ballon (26) of the catheter (24) are positioned inside the bladder (40),
b) inflating the ballon (26) and securing the ballon (26) against the bladder neck (28),
c) displacing the injection tube (12) inside the catheter (24), and
d) extending the injection tube (12) through an outlet opening (16) of the catheter (24) and inserting the injection tube (12) into the prostate (42), and
e) injecting a liquid including at least one anesthetic and adrenaline heated to a temperature of at least 37° C through the injection tube and through a plurality of openings (16) below an edge (15) of the hollow tip (14) into the prostate tissue.

13. The method as claimed in claim 12, further comprising
f) axially displacing the hollow tip (14) to another selected position in the tissue, and
g) repeating step e) of claim 12 at least once.

14. The method as claimed in claim 12, further comprising
a) retracting the injection tube (12) to a position inside the catheter (24),
b) rotating the catheter (24) at least 15 degrees,
c) extending the injection tube (12) through an outlet opening (16) of the catheter (24) and inserting the injection tube (12) into the prostate (42),
d) injecting a liquid including at least one anesthetic and adrenaline heated to a temperature of at least 37° C through the injection tube and through a plurality of openings (16) below an edge (15) of the hollow tip (14) into the prostate tissue.

15. The method as claimed in claim 14,
e) axially displacing the hollow tip (14) to another selected position in the tissue, and
f) repeating step e) of claim 12 at least once.
